# EUROPEAN PATENT SPECIFICATION

(11) **EP 4 400 519 B1**
(45) Date of publication and mention of the grant of the patent: **29.10.2025**
(21) Application number: 22909227.5
(22) Date of filing: 30.06.2022
(51) Int. Cl.: C07K 19/00, C12N 15/62, C12N 15/81, C12N 5/0783, C12N 1/19, A61K 38/17, A61K 39/395, A61P 31/04, A61P 35/00, A61P 35/02, A61P 31/14, A61P 31/16, A61P 31/20, C12R 1/84

(54) **BISPECIFIC NK CELL AGONIST, PREPARATION METHOD, AND APPLICATION**
BISPEZIFISCHER NK-ZELLAGONIST, HERSTELLUNGSVERFAHREN UND ANWENDUNG
AGONISTE BISPÉCIFIQUE DE CELLULES NK, PROCÉDÉ DE PRÉPARATION ET UTILISATION

(30) Priority: 20.12.2021 CN 202111566165
(43) Date of publication of application: 17.07.2024
(73) Proprietor: SHANGHAI NK CELLTECH CO., LTD., Shanghai 201318 (CN)
(72) Inventor: XIAO, Weihua, Shanghai 201318 (CN); TIAN, Zhigang, Shanghai 201318 (CN); CHEN, Minhua, Shanghai 201318 (CN)
(74) Representative: Dehns Germany Partnerschaft mbB
(86) International application number: PCT/CN2022/102876
(87) International publication number: WO 2023/115896

(56) References cited:
- WO-A1-2016/075278
- WO-A1-2021/119538
- WO-A2-2020/028572
- AU-A1- 2019 328 575
- CN-A- 110 461 357
- CN-A- 112 119 097
- CN-A- 114 349 869
- US-A1- 2019 382 507
- D. A. VALLERA ET AL: "IL15 Trispecific Killer Engagers (TriKE) Make Natural Killer Cells Specific to CD33+ Targets While Also Inducing Persistence, In Vivo Expansion, and Enhanced Function", CLINICAL CANCER RESEARCH, vol. 22, no. 14, 4 February 2016 (2016-02-04), US, pages 3440 - 3450, XP055509304, ISSN: 1078-0432, DOI: 10.1158/1078-0432.CCR-15-2710
- SCHMOHL J�RG U. ET AL: "Engineering of Anti-CD133 Trispecific Molecule Capable of Inducing NK Expansion and Driving Antibody-Dependent Cell-Mediated Cytotoxicity", vol. 49, no. 4, 15 October 2017 (2017-10-15), KR, pages 1140 - 1152, XP055946770, ISSN: 1598-2998, Retrieved from the Internet <URL:https://pdfs.semanticscholar.org/842e/15f6f5021cb6618e1e11ee4133833c0eefaa.pdf> DOI: 10.4143/crt.2016.491
- CARIAD CHESTER ET AL: "Immunotherapy targeting 4-1BB: mechanistic rationale, clinical results, and future strategies", BLOOD, ISSUE 832, vol. 131, no. 1, 4 January 2018 (2018-01-04), US, pages 49 - 57, XP055636174, ISSN: 0006-4971, DOI: 10.1182/blood-2017-06-741041
- CHRISTINA CLAUS ET AL: "Tumor-targeted 4-1BB agonists for combination with T cell bispecific antibodies as off-the-shelf therapy", SCIENCE TRANSLATIONAL MEDICINE, vol. 11, no. 496, 12 June 2019 (2019-06-12), pages eaav5989, XP055722436, ISSN: 1946-6234, DOI: 10.1126/scitranslmed.aav5989
- TANG TIAN, RAO WEI: "Progress of Natural Killer Cells and Their Application in Immunotherapy: from the Bench to Clinical", CHINA CANCER, vol. 26, no. 1, 5 January 2017 (2017-01-05), pages 44 - 52, XP093075725, ISSN: 1004-0242, DOI: 10.11735/j.issn.1004-0242.2017.01.A007
- ZHU HUANG, BLUM ROBERT H., BJORDAHL RYAN, GAIDAROVA SVETLANA, ROGERS PAUL, LEE TOM TONG, ABUJAROUR RAMZEY, BONELLO GREGORY B., WU : "Pluripotent stem cell–derived NK cells with high-affinity noncleavable CD16a mediate improved antitumor activity", BLOOD, vol. 135, no. 6, 6 February 2020 (2020-02-06), US , pages 399 - 410, XP055967123, ISSN: 0006-4971, DOI: 10.1182/blood.2019000621

## Description

### PRIORITY INFORMATION

This application claims priority to and the benefit of Patent Application No. 202111566165.1 filed on December 20, 2021 to the China National Intellectual Property Administration.

### TECHNICAL FIELD

The present disclosure relates to the field of biomedicine and specifically relates to a bispecific NK cell agonist, a preparation method, and a use.

### BACKGROUND

NK cells are the main effector cells of the innate immune system. Compared with T cells, NK cells can kill tumor cells and virus-infected cells without MHC restriction by using perforin and granzymes, as well as the related mechanisms without prior stimulation. NK cells express a large number of activating receptors, which can effectively recognize the stress ligands produced by tumor cells or infected cells, thereby effectively killing tumor cells, However, at the same time, tumor cells also secrete many immunosuppressive molecules to produce an immunosuppressive tumor microenvironment, such that the activity of NK cells in this environment and the exertion of anti-tumor ability are inhibited; thereby allowing NK cells to be in a state of exhaustion. Currently, there are also several strategies for restoring NK cell exhaustion in the tumor microenvironment, such as immune checkpoint blockade, activating antibodies, activating cytokines, etc., which can provide activation signals to NK cells or block the inhibitory signals in the tumor microenvironment, thereby restoring the normal activation state of NK cells, and exerting anti-tumor or anti-viral functions.

CD16A is an important class of activating receptors expressed on NK cells. When CD16A is activated by its ligand or antibody binding, it can induce a variety of cascade activation reactions, and release granzyme, perforin, inflammatory cytokines, and chemokines, which brings a strong activation effect and an enhancement of anti-tumor ability for NK cells. At the same time, the expression of 4-1BB receptor is temporarily upregulated. 4-1BB receptor is one of the members of the TNF receptor superfamily. 4-1BB is mainly expressed in the form of trimer on activated NK cells and T cells. Its ligand is 4-1BBL, which is also the only naturally occurring ligand found so far. When 4-1BBL binds to 4-1BB, it will activate 4-1BB receptor, and this process will bring a certain activation effect and an enhancement of the ADCC effect for NK cells. Vallera, D. A. *et al.* generated a bispecific killer engager (BiKE) containing single chain scFV against CD16 and CD33 to create an immunologic synapse between NK cells and CD33+ myeloid targets ("IL15 Trispecific Killer Engagers (TriKE) Make Natural Killer Cells Specific to CD33+ Targets While Also Inducing Persistence, In Vivo Expansion, and Enhanced Function." Clinical Cancer Research An Official Journal of the American Association for Cancer Research (2016):3440). WO2021119538A1 discloses compositions and methods for making and using engineered killer phagocytic cells for immunotherapy in cancer or infection by expressing a chimeric antigen receptor. WO2016075278A1 and US2019382507A1 both relate to novel TNF family ligand trimer-containing antigen binding molecules comprising (a) at least one moiety capable of specific binding to a target cell antigen and (b) a first and a second polypeptide that are linked to each other by a disulfide bond.

At present, there are few research reports on NK cell agonists, and the existing NK cell agonists have limited ability to activate NK cells, which needs to be further improved.

### SUMMARY

The present disclosure is intended to solve at least one of the technical problems in the related art to some extent. For this purpose, one object of the present disclosure is to provide a fused protein that uses a CD 16A receptor and a 4-1BB receptor as targets to obtain a new bispecific NK cell agonist medicament for activating an NK cell, enhancing the antivirus and antitumor capabilities of the NK cell, and has the potential effects of promoting NK cell proliferation and enhancing a T cell function. The invention is defined by the claims. The references to methods of treatment in the subsequent paragraphs of this description are to be interpreted as references to the compounds, pharmaceutical compositions and medicaments of the present invention for use in a method for treatment of the human (or animal) body by therapy.

To this end, in a first aspect, the present disclosure provides a fused protein. According to an embodiment of the present disclosure, the fused protein includes a CD16A single-chain antibody and a 4-1BBL extracellular region, wherein the CD16A single-chain antibody is linked to the 4-1BBL extracellular region, and wherein the 4-1BBL extracellular region at least includes three 4-1BBL extracellular domains, the three 4-1BBL extracellular domains being linked in sequence.

The inventors found that when a novel bispecific NK cell agonist medicament, scfv-CD16A/Trimer-4-1BBL, was designed by using the CD16A receptor and the 4-1BB receptor as targets, and selecting the single-chain CD16A antibody and the extracellular domain region of 4-1BBL, after the single-chain CD16A antibody recognizes the CD16A receptor, the NK cell agonist medicament recognizes the 4-1BB receptor through the extracellular domain region of 4-1BBL at the other end of the fused protein, resulting in increased proliferation of NK cells. The single-chain anti-CD16A antibody is linked to three 4-1BBL extracellular domains to construct a bispecific NK cell agonist medicament, which is used for activating NK cells, enhancing their antiviral and antitumor abilities, and has the effects of potentially promoting NK cell proliferation and enhancing T cell function. The scfv-CD16A/Trimer-4-1BBL fused protein provided by the present disclosure synergistically activates NK cells by binding to CD16A receptor and 4-1BB receptor, reverses the depletion state of NK cells, and enhances the anti-tumor ability of NK cells. The scfv-CD16A/Trimer-4-1BBL fused protein provided by the present disclosure can effectively amplify NK cells, improving NK cell purity and amplification factor.

According to an embodiment of the present disclosure, the fused protein further includes at least one of the following additional technical features.

Three 4-1BBL extracellular domains are linked to CD16A single-chain antibody to form scfv-CD16A-Trimer-4-1BBL. The scfv-CD16A-Trimer-4-1BBL has the activity of activating and amplifying NK cells in vitro, and the amplified NK cells have better cytotoxicity.

According to an embodiment of the present disclosure, the CD16A single-chain antibody and the 4-1BBL extracellular region are linked by a flexible linker.

According to an embodiment of the present disclosure, the three 4-1BBL extracellular domains are linked by a flexible linker.

According to an embodiment of the present disclosure, the fused protein is expressed by a non-mammalian cell expression system.

According to an embodiment of the present disclosure, the non-mammalian cell expression system includes at least one of a prokaryotic expression system and a low-level eukaryotic expression system.

According to an embodiment of the present disclosure, the non-mammalian cell expression system includes at least one of an *Escherichia coli* expression system, and a yeast expression system.

According to an embodiment of the present disclosure, the yeast expression system includes a *Pichia pastoris* expression system.

According to an embodiment of the present disclosure, the CD16A single-chain antibody includes a V_{H} region having an amino acid sequence as set forth in SEQ ID NO: 1 and a V_{L} region having an amino acid sequence as set forth in SEQ ID NO: 2.

According to an embodiment of the present disclosure, the V_{H} and V_{L} regions are linked by a first linker.

According to an embodiment of the present disclosure, the first linker is a flexible linker.

According to an embodiment of the present disclosure, the first linker has 10 to 20 amino acids in length.

According to an embodiment of the present disclosure, the first linker has an amino acid sequence as set forth in SEQ ID NO: 3.

The V_{H} region of the CD16A single-chain antibody has an amino acid sequence as set forth in the following SEQ ID NO: 1:

QVQLVQSGAEVKKPGESLKVSCKASGYTFTSYYMHWVRQAPGQGLEWMGIINP

SGGSTSYAQKFQGRVTMTRDTSTSTVYMELSSLRSEDTAVYYCARGSAYYYDFADY
WGQGTLVTVSS.

The V_{L} region of the CD16A single-chain antibody has an amino acid sequence as set forth in the following SEQ ID NO: 2:

SYVLTQPSSVSVAPGQTATISCGGHNIGSKNVHWYQQRPGQSPVLVIYQDNKRPS
GIPERFSGSNSGNTATLTISGTQAMDEADYYCQVWDNYSVLFGGGTKLTVL.

The the first linker has an amino acid sequence as set forth in the following SEQ ID NO: 3:
GGGGSGGGGSGGGGS.

According to an embodiment of the present disclosure, the V_{H} or V_{L} region has one end linked to the 4-1BBL extracellular region by a second linker.

According to an embodiment of the present disclosure, the second linker is a flexible linker.

According to an embodiment of the present disclosure, the second linker has 10 to 20 amino acids in length.

According to an embodiment of the present disclosure, the second linker has an amino acid sequence as set forth in SEQ ID NO: 4.

The second linker has an amino acid sequence as set forth in the following SEQ ID NO: 4:
GGGGSGGGGSGGGG.

According to an embodiment of the present disclosure, the 4-1BBL extracellular domain has an amino acid sequence as set forth in SEQ ID NO: 5.

The extracellular domain of 4-1BBL has an amino acid sequence as set forth in the following SEQ ID NO: 5:

REGPELSPDDPAGLLDLRQGMFAQLVAQNVLLIDGPLSWYSDPGLAGVSLTGGLS
YKEDTKELVVAKAGVYYVFFQLELRRVVAGEGSGSVSLALHLQPLRSAAGAAALALT
VDLPPASSEARNSAFGFQGRLLHLSAGQRLGVHLHTEARARHAWQLTQGATVLGLFR
VTPEIPAGLPSPRSE.

According to an embodiment of the present disclosure, the three 4-1BBL extracellular domains are linked in pairs by a third linker and a fourth linker, respectively, which are the same or different.

According to an embodiment of the present disclosure, the third linker and the fourth linker are both flexible linkers.

According to an embodiment of the present disclosure, the third linker and the fourth linker have 15 to 25 amino acids in length.

According to an embodiment of the present disclosure, the third linker has an amino acid sequence as set forth in SEQ ID NO: 6.

According to an embodiment of the present disclosure, the fourth linker has an amino acid sequence as set forth in SEQ ID NO: 7.

The third linker has an amino acid sequence as set forth in the following SEQ ID NO: 6:
GGGGSGGGGSGGGGSGGGGS.

The fourth linker has an amino acid sequence as set forth in the following SEQ ID NO: 7:
GGGGSGGGGSGGGGSGGGGS.

According to an embodiment of the present disclosure, the fused protein has an amino acid sequence as set forth in SEQ ID NO: 8.

The fused protein has an amino acid sequence as set forth in the following SEQ ID NO: 8:

QVQLVQSGAEVKKPGESLKVSCKASGYTFTSYYMHWVRQAPGQGLEWMGIINP
SGGSTSYAQKFQGRVTMTRDTSTSTVYMELSSLRSEDTAVYYCARGSAYYYDFADY
WGQGTLVTVSSGGGGSGGGGSGGGGSSYVLTQPSSVSVAPGQTATISCGGHNIGSKN
VHWYQQRPGQSPVLVIYQDNKRPSGIPERFSGSNSGNTATLTISGTQAMDEADYYCQV
WDNYSVLFGGGTKLTVLGGGGSGGGGSGGGGREGPELSPDDPAGLLDLRQGMFAQL
VAQNVLLIDGPLSWYSDPGLAGVSLTGGLSYKEDTKELVVAKAGVYYVFFQLELRRV
VAGEGSGSVSLALHLQPLRSAAGAAALALTVDLPPASSEARNSAFGFQGRLLHLSAG
QRLGVHLHTEARARHAWQLTQGATVLGLFRVTPEIPAGLPSPRSEGGGGSGGGGSGG
GGSGGGGSREGPELSPDDPAGLLDLRQGMFAQLVAQNVLLIDGPLSWYSDPGLAGVS

LTGGLSYKEDTKELVVAKAGVYYVFFQLELRRVVAGEGSGSVSLALHLQPLRSAAGA
AALALTVDLPPASSEARNSAFGFQGRLLHLSAGQRLGVHLHTEARARHAWQLTQGAT
VLGLFRVTPEIPAGLPSPRSEGGGGSGGGGSGGGGSGGGGSREGPELSPDDPAGLLDL
RQGMFAQLVAQNVLLIDGPLSWYSDPGLAGVSLTGGLSYKEDTKELVVAKAGVYYV
FFQLELRRVVAGEGSGSVSLALHLQPLRSAAGAAALALTVDLPPASSEARNSAFGFQG
RLLHLSAGQRLGVHLHTEARARHAWQLTQGATVLGLFRVTPEIPAGLPSPRSEHHHH
HH.

A second aspect of the present disclosure provides a nucleic acid. According to an embodiment of the present disclosure, the nucleic acid encodes the fused protein of the first aspect.

According to an embodiment of the present disclosure, the nucleic acid is a nucleic acid subjected to a codon optimization of the yeast expression system.

According to an embodiment of the present disclosure, the nucleic acid has a nucleotide sequence as set forth in SEQ ID NO: 10.

The nucleotide sequence encoding the above fused protein without codon optimization is set forth in the following SEQ ID NO: 9:

CAAGTTCAATTGGTTCAATCTGGTGCTGAGGTTAAGAAACCAGGTGAGTCTTT
GAAGGTTTCTTGTAAGGCTTCTGGTTATACTTTCACTTCTTACTATATGCATTGGGTT
AGACAAGCTCCAGGTCAAGGTTTGGAGTGGATGGGTATTATTAATCCATCTGGTGG
TTCTACTTCTTATGCTCAAAAGTTCCAAGGTAGAGTTACTATGACTAGAGATACTTC
TACTTCTACTGTTTATATGGAGTTGTCTTCTTTGAGATCTGAAGATACTGCTGTTTAT
TATTGTGCTAGAGGTTCTGCTTATTATTATGATTTCGCTGATTATTGGGGTCAAGGTA
CTTTGGTTACTGTCTCTTCTGGTGGTGGTGGTTCTGGTGGTGGTGGTTCTGGTGGT
GGTGGTTCCTCTTACGTCTTGACTCAACCATCTTCTGTCTCTGTTGCTCCAGGTCAA
ACTGCTACTATTTCTTGTGGTGGTCATAATATTGGTTCTAAGAATGTTCATTGGTATC
AACAAAGACCAGGTCAATCTCCAGTCTTGGTCATCTATCAAGATAACAAGAGACCA
TCTGGTATTCCAGAGAGATTCTCTGGTTCTAATTCTGGTAATACTGCTACTTTGACTA
TTTCTGGTACTCAAGCTATGGATGAAGCTGATTACTATTGTCAAGTTTGGGATAATTA
TTCTGTCTTGTTCGGTGGTGGTACTAAGTTGACTGTTTTGGGTGGTGGTGGTTCTgg

aGGTggcGGTTCTGGTGGTGGTCGCGAGGGTCCCGAGCTTTCGCCCGACGATC
CCGCCGGCCTCTTGGACCTGCGGCAGGGCATGTTTGCGCAGCTGGTGGCCCAAAA
TGTTCTGCTGATCGATGGGCCCCTGAGCTGGTACAGTGACCCAGGCCTGGCAGGC
GTGTCCCTGACGGGGGGCCTGAGCTACAAAGAGGACACGAAGGAGCTGGTGGTG
GCCAAGGCTGGAGTCTACTATGTCTTCTTTCAACTAGAGCTGCGGCGCGTGGTGGC
CGGCGAGGGCTCAGGCTCCGTTTCACTTGCGCTGCACCTGCAGCCACTGCGCTCT
GCTGCTGGGGCCGCCGCCCTGGCTTTGACCGTGGACCTGCCACCCGCCTCCTCCG
AGGCTCGGAACTCGGCCTTCGGTTTCCAGGGCCGCTTGCTGCACCTGAGTGCCGG
CCAGCGCCTGGGCGTCCATCTTCACACTGAGGCCAGGGCACGCCATGCCTGGCAG
CTTACCCAGGGCGCCACAGTCTTGGGACTCTTCCGGGTGACCCCCGAAATCCCAG
CCGGACTCCCTTCACCGAGGTCGGAAGGTGGTGGTGGTTCTggaGGTggcGGTTCTG
GTGGTGGTGGTTCTggaGGTggcGGTTCTCGCGAGGGTCCCGAGCTTTCGCCCGACG
ATCCCGCCGGCCTCTTGGACCTGCGGCAGGGCATGTTTGCGCAGCTGGTGGCCCA
AAATGTTCTGCTGATCGATGGGCCCCTGAGCTGGTACAGTGACCCAGGCCTGGCA
GGCGTGTCCCTGACGGGGGGCCTGAGCTACAAAGAGGACACGAAGGAGCTGGTG
GTGGCCAAGGCTGGAGTCTACTATGTCTTCTTTCAACTAGAGCTGCGGCGCGTGGT
GGCCGGCGAGGGCTCAGGCTCCGTTTCACTTGCGCTGCACCTGCAGCCACTGCGC
TCTGCTGCTGGGGCCGCCGCCCTGGCTTTGACCGTGGACCTGCCACCCGCCTCCTC
CGAGGCTCGGAACTCGGCCTTCGGTTTCCAGGGCCGCTTGCTGCACCTGAGTGCC
GGCCAGCGCCTGGGCGTCCATCTTCACACTGAGGCCAGGGCACGCCATGCCTGGC
AGCTTACCCAGGGCGCCACAGTCTTGGGACTCTTCCGGGTGACCCCCGAAATCCC
AGCCGGACTCCCTTCACCGAGGTCGGAAGGTGGTGGTGGTTCTggaGGTggcGGTTC
TGGTGGTGGTGGTTCTggaGGTggcGGTTCTCGCGAGGGTCCCGAGCTTTCGCCCGA
CGATCCCGCCGGCCTCTTGGACCTGCGGCAGGGCATGTTTGCGCAGCTGGTGGCC
CAAAATGTTCTGCTGATCGATGGGCCCCTGAGCTGGTACAGTGACCCAGGCCTGG
CAGGCGTGTCCCTGACGGGGGGCCTGAGCTACAAAGAGGACACGAAGGAGCTGG
TGGTGGCCAAGGCTGGAGTCTACTATGTCTTCTTTCAACTAGAGCTGCGGCGCGTG
GTGGCCGGCGAGGGCTCAGGCTCCGTTTCACTTGCGCTGCACCTGCAGCCACTGC
GCTCTGCTGCTGGGGCCGCCGCCCTGGCTTTGACCGTGGACCTGCCACCCGCCTC

CTCCGAGGCTCGGAACTCGGCCTTCGGTTTCCAGGGCCGCTTGCTGCACCTGAGT
GCCGGCCAGCGCCTGGGCGTCCATCTTCACACTGAGGCCAGGGCACGCCATGCCT
GGCAGCTTACCCAGGGCGCCACAGTCTTGGGACTCTTCCGGGTGACCCCCGAAAT
CCCAGCCGGACTCCCTTCACCGAGGTCGGAACATCATCACCATCACCAT.

The codon-optimized nucleotide sequence encoding the above fused protein is set forth in the following SEQ ID NO: 10:

CAAGTTCAGTTGGTGCAATCTGGTGCTGAGGTTAAGAAACCAGGTGAGTCAT
TGAAGGTTTCCTGTAAAGCCTCTGGTTATACTTTCACCTCATACTATATGCATTGGGT
TCGACAAGCTCCAGGTCAAGGTTTAGAGTGGATGGGTATAATTAATCCCTCTGGTG
GAAGCACTTCCTATGCTCAGAAGTTCCAAGGTCGGGTAACTATGACCCGTGATACA
AGCACATCTACAGTGTATATGGAGCTGTCGAGTTTAAGGAGTGAGGACACTGCTGT
TTATTACTGTGCGCGCGGTTCAGCATACTATTACGATTTCGCCGACTACTGGGGTCA
AGGAACTTTAGTAACAGTCTCTTCTGGAGGTGGAGGTTCTGGTGGAGGTGGATCT
GGTGGAGGTGGATCCTCTTACGTCTTGACTCAACCCTCTTCTGTCTCCGTAGCTCC
AGGTCAAACTGCCACAATTAGCTGTGGCGGGCATAATATAGGTAGTAAGAATGTTC
ATTGGTATCAACAGCGGCCAGGTCAGTCACCCGTTCTCGTCATCTACCAGGATAAC
AAGCGCCCCAGTGGTATTCCTGAGCGTTTCTCTGGTAGCAATTCGGGTAATACTGCT
ACGTTGACAATTAGCGGTACTCAAGCTATGGACGAGGCGGATTACTATTGCCAAGT
TTGGGATAATTATTCCGTCCTCTTCGGTGGGGGTACTAAGCTTACAGTGTTAGGTGG
AGGTGGATCTggaGGTggcGGTTCTGGTGGAGGTGGAAGAGAGGGACCAGAATTGT
CCCCAGATGACCCTGCTGGCTTATTGGACCTTAGACAAGGAATGTTTGCCCAGTTA
GTAGCTCAAAACGTTCTACTTATCGATGGTCCACTTTCATGGTATTCAGATCCAGGG
TTGGCGGGCGTCAGCCTGACCGGTGGACTGAGTTACAAAGAAGATACTAAGGAGT
TGGTTGTAGCTAAAGCAGGAGTTTACTATGTGTTTTTCCAACTCGAACTAAGACGA
GTTGTGGCCGGTGAAGGGTCGGGGTCTGTCTCCTTGGCTCTACACTTACAACCCCT
TCGTTCAGCTGCCGGAGCAGCAGCCCTGGCTCTGACAGTTGACTTGCCACCTGCA
TCCTCAGAAGCACGTAACAGTGCTTTTGGATTTCAAGGCAGATTGCTACATTTATCC
GCAGGGCAGAGACTCGGAGTGCACTTGCACACGGAGGCTAGAGCCAGGCATGCT
TGGCAGTTGACCCAGGGCGCTACTGTCTTGGGTCTGTTCAGAGTTACCCCTGAAAT

TCCTGCCGGACTTCCTTCGCCGAGGAGTGAAGGTGGAGGTGGTTCTGGTGGAGGT
GGATCTGGTGGAGGTGGATCTGGTGGTGGAGGTTCTAGAGAGGGACCAGAATTGT
CCCCAGATGACCCTGCTGGCTTATTGGACCTTAGACAAGGAATGTTTGCCCAGTTA
GTAGCTCAAAACGTTCTACTTATCGATGGTCCACTTTCATGGTATTCAGATCCAGGG
TTGGCGGGCGTCAGCCTGACCGGTGGACTGAGTTACAAAGAAGATACTAAGGAGT
TGGTTGTAGCTAAAGCAGGAGTTTACTATGTGTTTTTCCAACTCGAACTAAGACGA
GTTGTGGCCGGTGAAGGGTCGGGGTCTGTCTCCTTGGCTCTACACTTACAACCCCT
TCGTTCAGCTGCCGGAGCAGCAGCCCTGGCTCTGACAGTTGACTTGCCACCTGCA
TCCTCAGAAGCACGTAACAGTGCTTTTGGATTTCAAGGCAGATTGCTACATTTATCC
GCAGGGCAGAGACTCGGAGTGCACTTGCACACGGAGGCTAGAGCCAGGCATGCT
TGGCAGTTGACCCAGGGCGCTACTGTCTTGGGTCTGTTCAGAGTTACCCCTGAAAT
TCCTGCCGGACTTCCTTCGCCGAGGAGTGAAGGTGGAGGTGGATCTGGTGGAGGT
GGCTCTGGTGGCGGTGGTTCTGGTGGCGGTGGCTCTAGAGAGGGACCAGAATTGT
CCCCAGATGACCCTGCTGGCTTATTGGACCTTAGACAAGGAATGTTTGCCCAGTTA
GTAGCTCAAAACGTTCTACTTATCGATGGTCCACTTTCATGGTATTCAGATCCAGGG
TTGGCGGGCGTCAGCCTGACCGGTGGACTGAGTTACAAAGAAGATACTAAGGAGT
TGGTTGTAGCTAAAGCAGGAGTTTACTATGTGTTTTTCCAACTCGAACTAAGACGA
GTTGTGGCCGGTGAAGGGTCGGGGTCTGTCTCCTTGGCTCTACACTTACAACCCCT
TCGTTCAGCTGCCGGAGCAGCAGCCCTGGCTCTGACAGTTGACTTGCCACCTGCA
TCCTCAGAAGCACGTAACAGTGCTTTTGGATTTCAAGGCAGATTGCTACATTTATCC
GCAGGGCAGAGACTCGGAGTGCACTTGCACACGGAGGCTAGAGCCAGGCATGCT
TGGCAGTTGACCCAGGGCGCTACTGTCTTGGGTCTGTTCAGAGTTACCCCTGAAAT
TCCTGCCGGACTTCCTTCGCCGAGGAGTGAACATCATCATCACCATCAT.

A third aspect of the present disclosure provides an expression vector. According to an embodiment of the present disclosure, the expression vector includes the nucleic acid of the second aspect.

A fourth aspect of the disclosure provides a recombinant cell. According to an embodiment of the present disclosure, the recombinant cell carries the nucleic acid of the second aspect, the expression vector of the third aspect, or expresses the fused protein of the first aspect.

A fifth aspect of the disclosure provides the use of the fused protein of the first aspect, the nucleic acid of the second aspect, the expression vector of the third aspect, or the recombinant cell of the fourth aspect in the preparation of a medicament for treating or preventing a tumor or an inflammatory disease.

According to an embodiment of the present disclosure, the tumor includes at least one of: ovarian cancer, lung cancer, rectal cancer, breast cancer, or leukemia.

According to an embodiment of the present disclosure, the inflammatory disease includes a bacterial or virus infection.

According to an embodiment of the present disclosure, the bacterial infection includes at least one of *Klebsiella pneumoniae* infection, *Listeria monocytogenes* infection, or *Staphylococcus aureus* infection.

According to an embodiment of the present disclosure, the virus infection includes at least one of HPV infection, HBV infection, influenza virus infection, or coronavirus infection.

A sixth aspect of the present disclosure provides the use of the fused protein of the first aspect, the nucleic acid of the second aspect, the expression vector of the third aspect, or the recombinant cell of the fourth aspect in the culture and proliferation of NK cells in vitro .

According to an embodiment of the present disclosure, the NK cell is derived from a peripheral blood or an induced stem cell. According to an embodiment of the present disclosure, the NK cell includes at least one of: a NK cell derived from human peripheral blood mononuclear cell, a NK cell induced from human umbilical cord blood stem cell, a NK cell induced from human embryonic stem cell, or a NK cell induced from human iPSC.

A seventh aspect of the present disclosure provides a pharmaceutical composition. According to an embodiment of the present disclosure, the pharmaceutical composition includes the fused protein of the first aspect, the nucleic acid of the second aspect, the expression vector of the third aspect, or the recombinant cell of the fourth aspect.

According to an embodiment of the present disclosure, the pharmaceutical composition is used for treating or preventing a tumor or an inflammatory disease.

According to an embodiment of the present disclosure, the tumor includes at least one of: ovarian cancer, lung cancer, rectal cancer, breast cancer, and leukemia.

According to an embodiment of the present disclosure, the inflammatory disease includes a bacterial or virus infection.

According to an embodiment of the present disclosure, the bacterial infection includes at least one of *Klebsiella pneumoniae* infection, *Listeria monocytogenes* infection, or *Staphylococcus aureus* infection.

According to an embodiment of the present disclosure, the virus infection includes at least one of HPV infection, HBV infection, influenza virus infection, or coronavirus infection.

An eighth aspect of the disclosure provides a kit. According to an embodiment of the present disclosure, the kit includes the fused protein of the first aspect.

According to an embodiment of the present disclosure, the kit is used for the detection of CD16A and/or 4-1BB.

A ninth aspect of the disclosure provides a method of treating or preventing a tumor or an inflammatory disease. According to an embodiment of the present disclosure, the method includes administering to a subject suffering or suspected of having a tumor or an inflammatory disease at least one of:
the fused protein of the first aspect;
the nucleic acid of the second aspect;
the expression vector of the third aspect;
the recombinant cell of the fourth aspect;
the pharmaceutical composition of the seventh aspect.

According to an embodiment of the present disclosure, the tumor includes at least one of: ovarian cancer, lung cancer, rectal cancer, breast cancer, and leukemia.

According to an embodiment of the present disclosure, the inflammatory disease includes a bacterial or virus infection.

According to an embodiment of the present disclosure, the bacterial infection includes at least one of Klebsiella pneumoniae, Listeria monocytogenes, and Staphylococcus aureus infection.

According to an embodiment of the present disclosure, the virus infection includes at least one of HPV infection, HBV infection, influenza virus infection, or coronavirus infection.

A tenth aspect of the present disclosure provides the use of the fused protein of the first aspect, the nucleic acid of the second aspect, the expression vector of the third aspect, the recombinant cell of the fourth aspect, and the pharmaceutical composition of the seventh aspect in the treatment or prevention of a tumor or an inflammatory disease.

According to an embodiment of the present disclosure, the tumor includes at least one of: ovarian cancer, lung cancer, rectal cancer, breast cancer, and leukemia.

According to an embodiment of the present disclosure, the inflammatory disease includes a bacterial or virus infection.

According to an embodiment of the present disclosure, the bacterial infection includes at least one of Klebsiella pneumoniae, Listeria monocytogenes, and Staphylococcus aureus infection.

According to an embodiment of the present disclosure, the virus infection includes at least one of HPV infection, HBV infection, influenza virus infection, or coronavirus infection.

Additional aspects and advantages of the present disclosure will be shown in part in the description which follows and, in part, will be obvious from the description, or may be learned by practice of the present disclosure.

### BRIEF DESCRIPTION OF DRAWINGS

The foregoing and/or additional aspects and advantages of the present disclosure will become apparent and readily appreciated from the following description of the embodiments, taken in conjunction with the accompanying drawings of which:
FIG. 1 shows a schematic diagram of a protein expression vector and scfv-CD16A-Trimer-4-1BBL fused protein;
FIG. 2 shows the results of scfv-CD16A-Trimer-4-1BBL fused protein expression clone screening, wherein FIG. (A) shows the results of dot blot; (B) immunoblotting (Western Blot) results (anti-His tag antibodies);
FIG. 3 shows results of the fermentation expression of scfv-CD16A-Trimer-4-1BBL fused protein, wherein FIG. (A) shows the monitoring of fermentation process parameters and FIG. (B) shows the detection results of the target protein accumulation time point;
FIG. 4 shows the purification and physicochemical identification of scfv-CD16A-Trimer-4-1BBL fused protein, wherein FIG. (A) shows the detection result of SDS-PAGE for the purity of scfv-CD16A-Trimer-4-1BBL fused protein, FIG. (B) shows the detection result of dynamic light scattering for scfv-CD16A-Trimer-4-1BBL fused protein, and FIG. (C) shows the detection result of HPLC for scfv-CD16A-Trimer-4-1BBL fused protein;
FIG. 5 shows the detection results of in vitro activation of NK cells by scfv-CD16A-Trimer-4-1BBL fused protein;

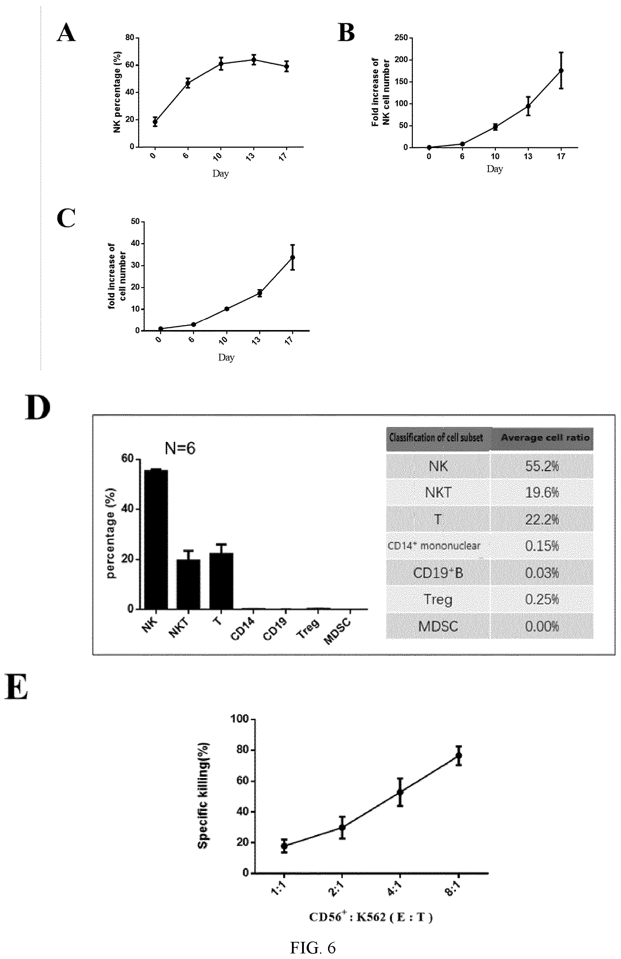

FIG. 6 shows the results of NK cell amplification induced by scfv-CD16A-Trimer-4-1BBL fused protein in vitro and the evaluation of the amplification effect, wherein FIG. (A) shows the detection result of NK purity after NK cell amplification induced by scfv-CD16A-Trimer-4-1BBL fused protein in vitro, and FIG. (B) shows the detection result of NK cell amplification fold induced by scfv-CD16A-Trimer-4-1BBL fused protein in vitro. FIG. (C) shows the detection result of total cell amplification fold after NK cell amplification induced by scfv-CD16A-Trimer-4-1BBL fused protein in vitro, FIG. (D) shows the detection result of cell subset after NK cell amplification induced by scfv-CD16A-Trimer-4-1BBL fused protein in vitro, and FIG. (E) shows the detection result of cell killing activity after NK cell amplification induced by scfv-CD16A-Trimer-4-1BBL fused protein in vitro.

### DESCRIPTION OF EMBODIMENTS

Embodiments of the present disclosure are described in detail below. The following described embodiments are exemplary and are merely for explaining the present disclosure and are not to be construed as limiting the present disclosure.

Furthermore, the terms "first" and "second" are used for descriptive purposes only and are not to be construed as indicating or implying relative importance or implicitly indicating the number of technical features indicated. Thus, a feature defined as "first" or "second" may explicitly or implicitly comprise at least one such feature. In the description of the present disclosure, the meaning of "plurality" is at least two, e.g. two, three, etc. unless specifically limited otherwise.

According to some specific embodiments of the present disclosure, the disclosure provides a fused protein including a CD16A single-chain antibody and a 4-1BBL extracellular region, wherein the CD16A single-chain antibody is linked to the 4-1BBL extracellular region, the 4-1BBL extracellular region including at least one to three 4-1BBL extracellular domains. For example, the 4-1BBL extracellular region includes three 4-1BBL extracellular domains, and the three 4-1BBL extracellular domains are linked in sequence to form a scfv-CD16A-Trimer-4-1BBL fused protein.

The fused protein provided by the present disclosure uses a CD16A receptor and a 4-1BB receptor as targets to obtain a new bispecific NK cell agonist medicament, i.e., scfv-CD16A-Trimer-4-1BBL; the medicament has the activity of activating and amplifying an NK cell in vitro, enhances the antivirus and antitumor capabilities of the NK cell, has the potential effects of promoting NK cell proliferation and enhancing a T cell function, and the amplified NK cell has good cytotoxicity.

As used herein, the term "antibody" is an immunoglobulin molecule capable of binding to a specific antigen, which comprises two light chains with lighter molecular weight and two heavy chains with heavier molecular weight, and the heavy chain (H chain) and light chain (L chain) are linked by a disulfide bond to form a tetrapeptide chain molecule. Among them, the amino-terminus (N-terminus) amino acid sequence of the peptide chain varies greatly and is called variable region (V-region), and the carboxy-terminus (C-terminus) is relatively stable and varies little and is called constant region (C-region). The V regions of the L and H chains are referred to as VL and VH, respectively, the C region of the L chain is CL and the C region of the H chain includes a CH1 region, a CH2 region, and a CH3 region.

In the present disclosure, the peripheral blood mononuclear cells (PBMC) are cells with a single nucleus in peripheral blood, including lymphocytes and monocytes.

According to some specific embodiments of the present disclosure, the first, second, third, and fourth linkage peptides are flexible polypeptides consisting of glycine and serine.

According to some specific embodiments of the present disclosure, the pharmaceutical composition provided by the present disclosure further includes a pharmaceutically acceptable carrier, including any solvent, solid excipient, diluent, binder, disintegrant, or other liquid excipient, dispersing agent, flavoring or suspending agent, surface active agent, isotonic agent, thickening agent, emulsifying agent, preservative, solid binder, glidant or lubricant, and the like, suitable for the particular desired dosage form. Except insofar as any conventional adjuvant is incompatible with the fused proteins of the present disclosure, such as by producing any undesirable biological effect or interacting in a deleterious manner with any other component(s) of the pharmaceutically acceptable composition, its use is contemplated to be within the scope of this disclosure.

For example, the fused proteins of the present disclosure can be incorporated into the pharmaceutical compositions suitable for parenteral administration (e.g. intravenous, subcutaneous, intraperitoneal, intramuscular). The pharmaceutical composition can be prepared in various forms. For example, liquid, semisolid, and solid dosage forms, and the like, including, but are not limited to, liquid solutions (e.g. injection and infusion solution), dispersions or suspensions, tablets, pills, powders, liposomes, and suppositories. The fused protein can be administered by intravenous injection or intramuscular or subcutaneous injection.

As used herein, the terms "treat" and "prevent", and words derived therefrom do not necessarily imply 100% or complete treatment or prevention. Rather, there are varying degrees of treatment or prevention that would be considered by one of ordinary skill in the art to be of a potential benefit or therapeutic effect. Moreover, the treatment or prevention provided by the present disclosure can include the treatment or prevention of one or more conditions or symptoms of a disease being treated or prevented, such as cancer. Additionally, for purposes herein, "prevent" can encompass delaying the onset of a disease or a symptom or condition thereof.

Examples of the present disclosure are described in detail below. The following described examples are exemplary and are merely for explaining the present disclosure and are not to be construed as limiting the present disclosure. Where specific techniques or conditions are not specified in the examples, they are performed according to techniques or conditions described in the literature in the art or according to the product description. The reagents or instruments used are conventional products that can be obtained commercially without indicating the manufacturer.

### Example 1. Construction of a fused protein expression vector of the bispecific NK cell agonist scfv-CD16A-Trimer-4-1BBL

### 1. Obtaining scfv-CD16A-Trimer-4-1BBL fusion gene

The sequence information about scfv-CD16A (scfv-CD16A includes V_{H} and V_{L}, and the V_{H} and V_{L} are linked by linker 1, wherein the amino acid sequence of V_{H} is as set forth in SEQ ID NO: 1, the nucleotide sequence of V_{L} is as set forth in SEQ ID NO: 2, and the nucleotide sequence of linker 1 is as set forth in SEQ ID NO: 3) was obtained, and the sequence of 4-1BBL extracellular segment 71-254(as set forth in SEQ ID NO: 5) was obtained from NCBI; scfv-CD16A was linked to three 4-1BBL extracellular domains by linker 2 (as set forth in SEQ ID NO: 4), and three 4-1BBL extracellular domains were linked by linker 3 (as set forth in SEQ ID NO: 6) and linker 4 (as set forth in SEQ ID NO: 7) in sequence. The original scfv-CD16A-Trimer-4-1BBL fusion gene obtained without codon optimization is as set forth in SEQ ID NO: 9.

The original scfv-CD16A-Trimer-4-1BBL fusion gene sequence was adjusted and modified, *Pichia pastoris* codon optimization was performed and two enzymatic cleavage sites of Xhol and Not I were added at both ends. The optimized and modified gene sequence is as set forth in SEQ ID NO: 10; the gene sequence was then subjected to whole gene synthesis to obtain the scfv-CD16A-Trimer-4-1BBL fused protein gene sequence.

2. Construction of Pichia pastoris expression vector of scfv-CD16A-Trimer-4-1BBL fused protein

The gene sequence of the scfv-CD16A-Trimer-4-1BBL fused protein obtained in the above step and *Pichia pastoris* expression plasmid PGAPzα-rDNA-NTS were both double-digested with restriction endonucleases i.e., Xhol and Not I. The digested scfv-CD16A-Trimer-4-1BBL fused protein gene sequence product gene was recovered using a PCR product recovery kit (Axygen), and the digested plasmid was recovered using a DNA gel recovery kit (Axygen). The operation procedures can refer to the kit instructions. The recovered digested fused protein gene was linked with the digested vector using T4 DNA ligase. The enzyme-linked product was transformed into *E. coli* TOP10 competent cells. The transformation procedure was as follows: 10 µl of the enzyme-linked product was added into 100 µL of *E. coli* competent cells, mixed well, allowed to stand on ice for 15 to 30 min, and bounced evenly every 5 min. This was followed by heat shock at 42°C for 90 s, standing on ice for 3 to 5 min, 400 µl of LB liquid culture medium without resistance was added, the mixture was revived and cultured at 37°C on a shaker for 45 min, and plated on Zeocin resistant plates.

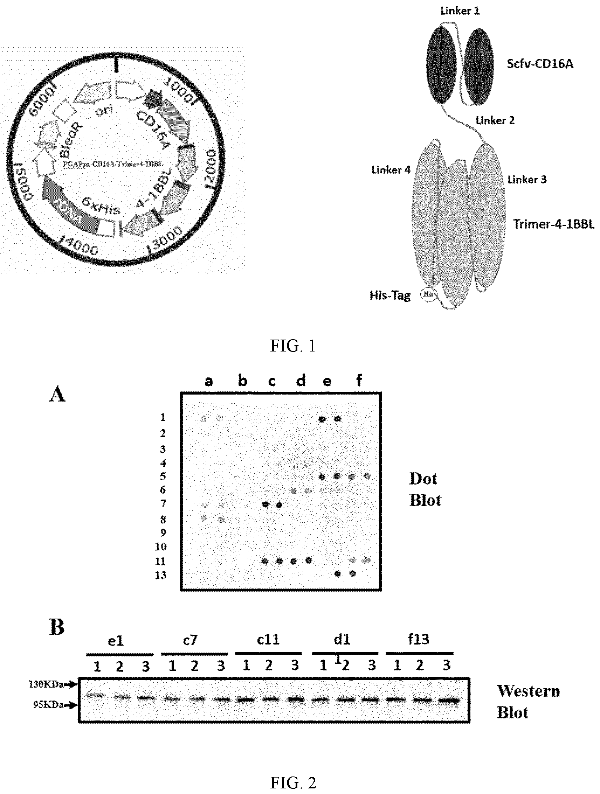

After the clonal colonies grew on the plate, a single colony was selected and cloned into a shake tube containing LZ liquid culture medium (LB culture medium with Zeocin resistance), and cultured at 37 for 6 to 8 h. Then, the bacterial solution was used as a template for PCR identification, and the obtained positive clone is named as PGAP-zα-scfv-CD16A-Trimer-4-1BBL (protein expression vector is as shown in FIG. 1). The bacterial solution corresponding to the positive clone was extracted with the plasmid small-scale extraction kit and sent to General Biological and GENEWIZ for sequencing.

After sequencing, the sequencing results were consistent with the theoretical expected result, and the clone was constructed correctly.

### Example 2. Expression and purification of the fused protein of the bispecific NK cell agonist scfv-CD16A-Trimer-4-1BBL

### 1. Fused protein expression

The positive clone constructed in Example 1 was amplified and cultured using 50 mL of LZ liquid culture medium, and a medium amount of plasmid PGAP-zα-scfv-CD16A-Trimer-4-1BBL was extracted using a kit (Axygen Company). The expression plasmid PGAPzα-scfv-CD16A-Trimer-4-1BBL prepared in the previous step was linearized with endonuclease Spe I and recovered by ethanol precipitation.

Ethanol precipitation steps: 1) Two times the volume of anhydrous ethanol and 0.1 times the volume of 3 M sodium acetate were added to the enzyme digestion reaction system and precipitated at -20°C for more than 2 h. 2) The mixture was centrifuged at 12,000 g for 10 min and the supernatant was discarded. 3) The precipitate was resuspended in 300 µL of 70% ethanol, centrifuged at 12,000 g for 10 min and the supernatant was discarded. 4) The mixture was dried at 37°C, and re-suspended with deionized water, the concentration was determined and adjusted to 0.5 to 1.0 µg/µL.

Preparation of yeast competent: preparing steps: 1) the well-frozen *Pichia pastoris* X33 strain was streaked on a YPD plate and cultured at 30°C in a yeast incubator; 2) after the colonies grew to about 1 mm in diameter, they were picked and cultured in liquid 4 mL of YPD culture medium on a yeast shaker at 30°C; 3) cultured for 24 to 48 h. 1 mL of the broth was inoculated into 50 mL of fresh YPD culture medium; 4) when the OD600 value reached 1 to 1.5, the bacterial solution was transfered to a 50 mL centrifuge tube, centrifuged at 1,500 g for 5 min at 4°C, and the supernatant was discarded; 5) resuspended in 50 mL of ice water, and centrifuged at 1,500g for 5 min at 4°C, the supernatant was discarded and repeated once; 6) resuspended in 50 mL of 1 M cold sorbitol, centrifuged at 1,500 g for 5 min at 4°C, the supernatant was discarded and repeated once; and 7) resuspended in 500 µL of 1 M cold sorbitol.

Electroporation steps: 100 µL of the above competent cells was taken in a sterile precooled electroporation cuvette, 10 µL of linearized plasmid (5 to 10 µg) was added, and mixed well. The parameters of the electroporator were set as 2000 V, 200 Ω, 25 µF. Electroporation was performed, after electroporation, 1 mL of 1 M cold sorbitol was immediately added into the electroporation cuvette. The mixture was allowed to stand on ice for 5 to 10 min, then the whole bacterial solution was transferred into a 50 mL centrifuge tube filled with 1 mL of YPD, to recover at 30°C and 225 rpm for 2 h. At the end of recovery, 100 to 1000 µL of the above bacterial solution was coated onto a YPDZ (plate containing Zeocin resistance) plate, and the plate was placed in a constant temperature yeast incubator at 30°C for culture.

Positive expression clone selection: after the yeast colonies grew out from the above-mentioned YPDZ plate, several colonies were picked and cultured in 2 mL of BMGY culture medium at a constant temperature of 28°C on a yeast shaker until the bacterial solution became milky. The 1 mL of bacteria solution was frozen and preserved, after centrifuging at 12,000 g for 5 min, the supernatant of the remaining 1 mL of solution was taken for identification and primary screening using Dot blot with Anti-His tag antibody (results were shown in FIG. 2). The yeast strains with relatively high expression were picked out, and then the yeast strains with relatively high expression (e1, c7, c11, d1, f13) were coated on the YPD plate containing zeocin. After the clones were grown, several clones were selected to shake the bacteria in the above way and the supernatant of the bacteria was obtained, and the Western Blot identification and analysis was carried out with Anti-His tag antibody. The strain with high expression was screened, and the remaining 1 mL solution of the corresponding strain was frozen and preserved; the high expression strain (f13-3) is a *Pichia pastoris* strain expressing scfv-CD16A-Trimer-4-1BBL complex protein.

### 2. Fermentation, purification, and identification of fused proteins

Culturing expression strain: fermentation of the recombinant strains was carried out according to the fermentation instruction manual of Invitrogen. The screened high-expression strains were inoculated into a shake tube containing 4 mL of YPD culture medium, and the mixture was cultured at 30°C for 24 to 48 h on a shaker until the OD600 was 2 to 6, i.e. the fermentation primary seed solution. 1 mL of the primary fermentation seed solution was transferred to an Erlenmeyer flask containing 200 mL of BMGY culture medium, and the mixture was cultured at 30°C for 12 to 24 h on a shaker to obtain a secondary fermentation seed solution. 200 mL of the secondary seed solution was totally inoculated into a fermentor containing 6 L of BMGY culture medium. Setting parameters: culture temperature was 30°C, pH was 6.0, dissolved oxygen and rotation speed were monitored, and fermentation culture was started. BMGY culture medium was used in the growth stage of bacteria. When the dissolved oxygen rose rapidly, the basal glycerol was consumed in the culture medium. Glycerol feeding was started. The glycerol feeding rate was as follows: 70 mL/h, the fermentation temperature was adjusted to 25°C, and continued until the end of fermentation.

Clarifying culture solution: after completion of the fermentation, the mixture was centrifuged at 10,000 g for 30 min, and the supernatant was collected and clarified by filtration through 0.22 um and 500 kDa microfiltration membranes, after which the pH was adjusted to 7.4.

Protein capturing: the recombinant fused protein was captured using an affinity chromatography column, a nickel column. The specific steps were: 1) the column was flushed with deionized water in 5 column volumes; 2) the column was flushed with PBS in three column volumes; 3) loading; 4) after loading, the column was flushed with PBS in three column volumes; 5) washing impurity: the column was flushed with a gradient of PBS solution containing 10 mM, 20 mM, 30 mM, and 40 mM imidazole in 1 to1.5 column volumes per concentration; and 6) the target protein was eluted, the eluate was a PBS solution containing 200 mM imidazole, and the collected eluent was concentrated and used for subsequent purification.

Fine purifying: the Superdex200 molecular sieve was equilibrated with PBS buffer using AKTA PURE 25, and after equilibration, the neutral eluate was loaded, followed by washing with PBS buffer and the eluted sample was collected. Subsequently, the sample was concentrated and subjected to Superdex75 molecular sieves in the same manner to obtain scfv-CD16A-Trimer-4-1BBL recombinant protein.

Identifying recombinant proteins: the purified protein was identified by SDS-PAGE, HPLC, and dynamic light scattering.

FIG. 2 shows that a yeast expression strain of the fused protein was obtained by YPDZ resistance culture plate selection. Using Dot blot for preliminary screening, some clones with relatively high expression levels can be obtained preliminarily, followed by further confirmation and comparison by Western Blot, the yeast expression clones with higher expression levels can be selected for subsequent protein expression purification.

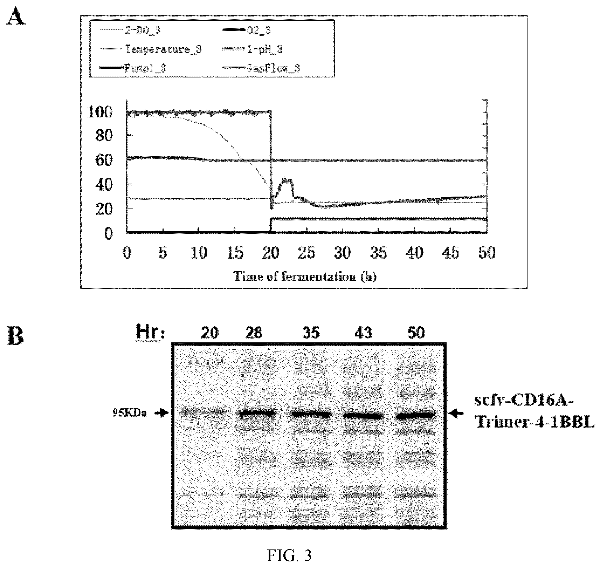

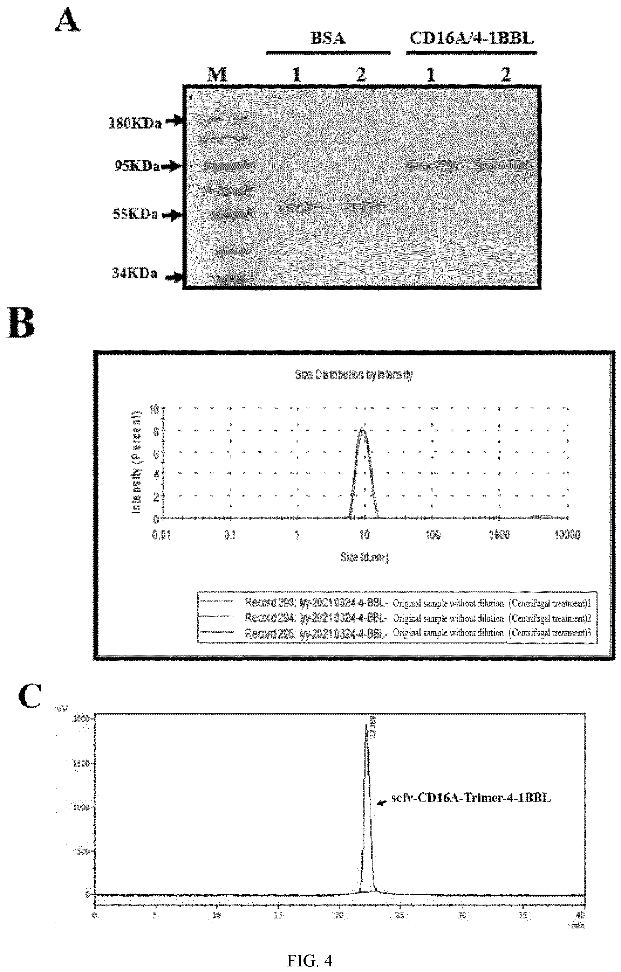

FIG. 3 shows the monitoring of various parameters of the fermentation process. The whole fermentation process lasted for 50 h, and the fermentation process was stable and easy to repeat. As shown in FIG. 3A, the glycerol feeding restriction culture was performed from the end of the basal glycerol culture stage to induce protein expression (20 h), and protein accumulation continued throughout the process, with little difference in protein accumulation between 43 to 50 h, and the end of fermentation was determined (FIG. 3B shows the amount of scfv-CD16A-Trimer-4-1BBL recombinant protein in the fermentation supernatant at 20 h, 28 h, 35 h, 43 h and 50 h of fermentation, respectively). Through centrifugation and two-step purification, the protein purity was more than 95% as identified by SDS-PAGE, HPLC (FIG. 4A and FIG. 4C), and the molecular weight of scfv-CD16A-Trimer-4-1BBL protein is about 95 kDa, which is consistent with the expectation. Through dynamic light scattering identification, the molecular diameter of scfv-CD16A-Trimer-4-1BBL was about 10 nm and the intermolecular homogeneity is good (FIG. 4B).

### Example 3. Verification of the NK cell activation function induced in vitro by the fused protein of the bispecific NK cell agonist scfv-CD16A-Trimer-4-1BBL

Human PBMC was isolated by Ficoll density gradient centrifugation. The isolated PBMCs were diluted with RPMI 1640 containing 10% FBS to 1.0 x 10⁶/mL. 10 mL of diluted cells was added to the T25 culture flask. After 12 h of culture, scfv-CD16A-Trimer-4-1BBL fused protein was added to the culture flask at a final concentration of 20 nM. After 24 h of culture, the phenotype changes of NK cells were detected by flow cytometer.

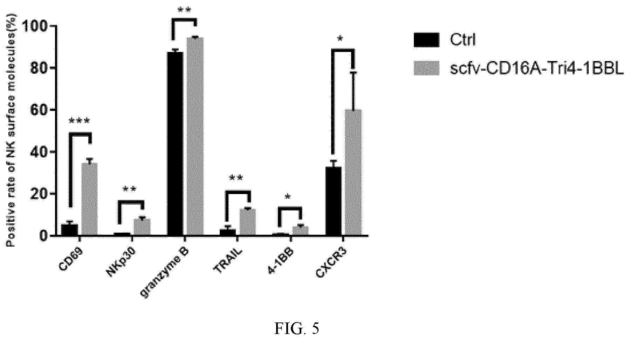

Results were as shown in FIG. 5, compared with control group PBS, in the experimental group added with scfv-CD16A-Trimer-4-1BBL fused protein, the expression of major activating molecules such as CD69, NKp30, killing molecules 4-1BB, TRAIL, Granzyme B and chemotactic molecule CX3CR1 on the surface of NK cells was significantly up-regulated, so it was judged that NK cells were effectively activated by scfv-CD16A-Trimer-4-1BBL fused protein.

### Example 4. NK cell amplification induced in vitro by fused protein of the bispecific NK cell agonist scfv-CD16A-Trimer-4-1BBL and evaluation of the amplification effect

### 1. Purity and quantity detection of NK cells amplified by fused protein:

1) The fused protein was used to coat the T75 culture flask 24 h in advance, 7.5 mL of coating solution was taken from each flask, to cover the bottom of the flask body. Coating overnight in a refrigerator at 4°C.
2) After PBMC cells were isolated from the blood samples, they were counted (Mindray counter) and the percentage of each cell subset in PBMC cells was recorded. Inoculating at a density of 1.5*10⁶/ml (20 ml culture volume). The total number of cells was 30*10⁶. This was Day 0. Meanwhile, the KBM581 culture medium without scfv-CD16A-Trimer-4-1BBL was supplemented with 1% IL2 (1000 IU/ml) and 5% of the corresponding PBMC autologous plasma.
3) On Day 3, the IL2 factor at a volume of 1% of the corresponding culture volume was supplemented.
4) On Day 5, the color of the culture medium and cell adherence state at the bottom of the T75 flask body were observed, 5 to 10 ml of KBM581 culture medium containing scfv-CD16A-Trimer-4-1BBL was supplemented as needed, and IL2 (1000 IU/ml) factor at a volume of 1% of the supplemented solution was added.
5) On Day 6, samples were taken for counting for cell passage and amplified culture (1.3*10⁶/ml). After staining and labeling (CD3/CD45/CD56), the percentage of NK cell subset and the percentage of CD56+ cell subset were detected by flow cytometer, and the total cell number was calculated and the NK cell number was calculated according to the results of flow purity detection. Basal culture medium containing scfv-CD16A-Trimer-4-1BBL was used for cell passage and amplified culture from Day 5, which was supplemented with 1% IL2 and 2% autologous plasma.
6) On Day 7, the color of the culture medium and the adherent growth of the cells on the flask body were observed, KBM581 culture medium supplementation was performed as needed and IL2 factor at a volume of 1% of fluid supplementation was added.
7) On Day 8, samples were counted. Cell passage and amplification were performed as needed (T75 or T175 flasks, 1.4*10⁶/mL) and supplemented with 1% IL2 factor, and 2% autologous plasma in culture medium.
8) On Day 9, the color of the culture medium and the adherent growth of the cells on the flask body were observed, and fluid supplementation was performed as needed (with the addition of IL2 factor at a volume of 1% of fluid supplementation).
9) On Day 10, samples were taken for counting for cell passage and amplified culture (1.5*10⁶/ml). After staining and labeling (CD3/CD45/CD56), the percentage of NK cell subset and the percentage of CD56+ cell subset were detected by flow cytometer, and the total cell number was calculated and the NK cell number was calculated according to the results of flow purity detection.
10) On Day 12, the color of the culture medium and the adherent growth of the cells on the flask body were observed, and fluid supplementation was performed as needed (with the addition of IL2 factor at a volume of 1% of fluid supplementation).
11) On Day 13, samples were taken for counting for cell passage and amplified culture (1.5*10⁶/mL). After staining and labeling (CD3/CD45/CD56), the percentage of NK cell subset and the percentage of CD56+ cell subset were detected by flow cytometer, and the total cell number was calculated and the NK cell number was calculated according to the results of flow purity detection.
12) On Day 15, samples were counted. Cell passage and amplification were performed as needed (T175 flasks or culture bags, 1.5*10⁶/mL) and supplemented with 1% IL2 factor in the culture medium.
13) On Day 17, samples were taken for counting, after staining and labeling (CD3/CD45/CD56), the percentage of NK cell subset and the percentage of CD56+ cell subset were detected by flow cytometer, and the total cell number was calculated and the NK cell number was calculated according to the results of flow purity detection. NK cell proportion and number are plotted as a function of time by NK purity and cell number at Day 0, 6, 10, 13, 17.

### 2. Analysis of the subset of PBMC-derived NK cells amplified by fused protein

1) Inducing intracellular molecular labeled cells with monensin: 4 x 10⁶ cultured cells (2 mL) were taken and placed in a 6-well plate, 10 mL of monensin (0.5 µg/µl) was added, cells were incubated at 37°C, 5% CO₂ constant temperature incubator for induction for 4 h.
2) Preparing single cell suspension: 9.0 x 10⁶ cultured cells from the surface molecular labeled cells were taken into a 15 mL centrifuge tube, centrifuged at 400 g for 8 min to collect the cells, washed twice with 10 ml of 1X PBS, and finally resuspended into a single cell suspension with 0.81 mL of 1X PBS. After induction of intracellular molecular labeled cells with monensin, the cells were washed twice with 5 mL of 1X PBS and finally resuspended with 180 µL of 1X PBS into a single cell suspension.
3) Blocking: 90 µL of mouse serum was added to the surface molecular labeled cells, mixing well, 20 µL of mouse serum was added to the intracellular molecular labeled cells, mixing well, and allowing to stand at room temperature for 15 to 30 min.
4) Labeling antibodies: after blocking, the surface molecular labeled cells were divided into 9 flow tubes at 0.09 mL/tube. If there are multiple batches of cells to be detected at the same time, the cells in tubes 1 to 8 can be labeled by mixing several batches of cells in equal amounts, and the amount of cells in each tube was: 0.8 to 1 x 10⁶ cells, Sample tube 1 was a surface molecular labeled, and the amount of cells was: 0.8 to 1 x 10⁶. Sample tube 2 was an intracellular molecular marker, and the amount of cells was: 3 to 4 x 10⁶ cells, which were added with the corresponding fluorescently labeled antibody, mixed well and the mixture was allowed to stand for 30 min at 4°C, protected from light (mixed well every 15 min during the process).

**Table 1: Sample tube for cell surface molecule detection and experimental operation table**

| No. | Groups | Fluorescent labeled antibody | Dosage |
|---|---|---|---|
| 1 | Blank control tube | / | / |
| | | Mouse IgG1-Alexa Flour 647 | 0.5 µl |
| | | Mouse IgG1-percp cy5.5 | 0.5 µl |
| | | Mouse IgG1- BV785 | 0.5 µl |
| 3 | Single-labeled control tube | CD3- Percpcy5.5 | 0.5 µl |
| 4 | | CD56- BV785 | 0.5 µl |
| 5 | | DAPI | 5 µl |
| 6 | | BV510-CD19 | 1 µl |
| 8 | | BV650-CD14 | 1 µl |
| Sample tube | | CD3-Percp cy5.5 | 0.5 µl |
| | | CD56- BV785 | 0.5 µl |
| | | CD14-BV650 | 1 µl |
| | | CD19-BV510 | 1 µl |

5) Washing: 1 mL of 1X PBS was added to each tube of surface molecular labeled cells and the cells were harvested by centrifuging at 4°C, 400 g for 8 min. The cells were washed 2 times with 1 ml of 1X PBS. Finally, 200 µL of PBS was added to each tube to resuspend the labeled cells, 5 µL of DAPI (50 µg/mL, 40x) was added to the experimental group, and the tube was transferred to the machine for detection.
6) Testing: the flow cytometer was calibrated and adjusted according to the instructions for use. The blank control tube samples were used to adjust the forward and side scatter voltages. A single-labeled control sample was used to adjust the fluorescence compensation of each channel. After the cell gates were delineated during the assay, 1 x 10⁴ cells were collected in each sample gate.

### 3. Detection of killing activity of NK cells amplified by fused protein

### Preparation of Target cell (K562) suspension

1) Cell counting: the cultured K562 cells were suspended in 1640 culture medium containing 0.5% FBS (the culture medium used for target cells), counted and the cell density was adjusted to 1 x 10⁶/mL.
2) CFSE staining: CFSE (working concentration: 5 µM) was added to the cell suspension, and the cells were pipetted and mixed well with 1 mL pipette immediately, followed by vortex well, incubating 15 min in a 37°C incubator protected from light, and taking out every 5min to vortex well.
3) Stop staining: the staining was stopped by adding 5 volumes of complete culture medium precooled at 4°C (the culture medium used for target cells) and incubated on ice for 5 min,followed by centrifuging at 140 g, 4°C, 5 min;
4) Washing cells: complete culture medium precooled at 4°C (the culture medium used for target cells) was used to re-suspend the cells, and the cells were centrifuged at 140 g, 4°C for 5 min. The washing was repeated twice.
5) Counting: the cells were resuspended and counted, and the cell density was adjusted to 2 x 10⁵/mL;
6) Plating: 100 µl of K562 suspension was added into each well of the 96-well round bottom plate so that the final cell number in each well is 20000 cells/well.

### Preparation and Addition of NK Cells

7) NK cells a suitable were prepared at a suitable density;
8) The E-Plate 16 was taken and placed on a super clean bench;
9) 100 µl of NK cell suspension at different CD56+ : K562 effect target ratios (1:1, 2:1, 4:1, 8:1) was added into the culture plate. In addition, three groups of controls were provided, i.e., target cells stained with CFSE only (natural mortality of target cells was detected); only effector cells (demonstrating that effector cells do not contain CFSE non-specific staining); target cells + Tween-20 (as positive control for apoptosis of target cells).
10) Co-incubation, NK cells were added to each well in a pre-designed order. The effect target cells were contacted thoroughly by centrifugation at 120 g, at room temperature for 2 min. The cells were returned for incubating in a 37°C incubator for 4 h.
11) At the end of incubation, 5 µL of PI was added, mixed well, incubated for 5 min protected from light, and detected on the machine.
12) Analysis of results: percentage of killing = [(death rate (%) of target cells in experimental group-death rate (%) of target cells)/(100%-natural death rate (%) of target cells)] x 100%).

### 4. Result analysis

As shown in FIG. 6A to FIG. 6C, the amplification of NK cells derived from PBMC by using scfv-CD16A-Trimer-4-1BBL can effectively amplify NK cells and improve the purity and amplification factor of NK cells, and the highest proportion of NK cells in the whole culture process can reach about 60%.

As shown in FIG. 6D, cell subset analysis of the amplified cells showed that in the PBMC cells treated and amplified with scfv-CD16A-Trimer-4-1BBL, the proportion of NK cells was up to 55%, the proportion of NKT was about 20%, and the proportion of total CD56 positive cells was about 75%, and the remaining cell subsets were relatively low in the amplified PBMC cells.

As shown in FIG. 6E, the cytotoxicity of the amplified cells was evaluated, and the amplified cells could effectively kill K562 cells.

In conclusion, the results demonstrated that scfv-CD16A-Trimer-4-1BBL had the activity of activating and amplifying NK cells *in vitro,* and the amplified NK cells had better cytotoxicity.

In the description of this specification, references to descriptions of the terms "one embodiment", "some embodiments", "examples", "specific examples", or "some examples", etc. mean that a particular feature, structure, material, or characteristic described in connection with the embodiment or example is included in at least one embodiment or example of the present disclosure. In this specification, schematic representations of the above terms are not necessarily directed to the same embodiment or example. Furthermore, the particular features, structures, materials, or characteristics described may be combined in any suitable manner in any one or more embodiments or examples. Furthermore, combinations and combinations of the various embodiments or examples and features of the various embodiments or examples described in this specification can be made by those skilled in the art without departing from the scope of the present disclosure.

## Claims

1. A fused protein, comprising a CD16A single-chain antibody and a 4-1BBL extracellular region, wherein the CD16A single-chain antibody is linked to the 4-1BBL extracellular region, **characterized in that**, the 4-1BBL extracellular region comprises three 4-1BBL extracellular domains, the three 4-1BBL extracellular domains being linked in sequence.

2. The fused protein according to claim 1, wherein the CD16A single-chain antibody and the 4-1BBL extracellular region are linked by a flexible linker;
optionally, the three 4-1BBL extracellular domains are linked by the flexible linker.

3. The fused protein according to claim 1 or 2, wherein the fused protein is expressed by a non-mammalian cell expression system;
optionally, the non-mammalian cell expression system comprises at least one of a prokaryotic expression system and a low-level eukaryotic expression system;
optionally, the non-mammalian cell expression system comprises at least one of an *Escherichia coli* expression system and a yeast expression system; and
optionally, the yeast expression system comprises a *Pichia pastoris* expression system.

4. The fused protein according to any one of claims 1 to 3, wherein:
the CD16A single-chain antibody comprises a V_{H} region and a V_{L} region;
the V_{H} region has an amino acid sequence as set forth in SEQ ID NO: 1;
the V_{L} region has an amino acid sequence as set forth in SEQ ID NO: 2;
optionally, the V_{H} and V_{L} regions are linked by a first linker;
optionally, the first linker is a flexible linker;
optionally, the first linker has 10 to 20 amino acids in length;
optionally, the first linker has an amino acid sequence as set forth in SEQ ID NO: 3.

5. The fused protein according to claim 4, wherein the V_{H} region or V_{L} region has one end linked to the 4-1BBL extracellular region by a second linker;
optionally, the second linker is a flexible linker;
optionally, the second linker has 10 to 20 amino acids in length;
optionally, the second linker has an amino acid sequence as set forth in SEQ ID NO: 4.

6. The fused protein according to any one of claims 1 to 5, wherein the three 4-1BBL extracellular domains has each an amino acid sequence as set forth in SEQ ID NO: 5.

7. The fused protein according to any one of claims 1 to 6, wherein the three 4-1BBL extracellular domains are linked in pairs by a third linker and a fourth linker, respectively, the third linker and the fourth linker being the same or different;
optionally, the third linker and the fourth linker are both flexible linkers;
optionally, the third linker and the fourth linker have 15 to 25 amino acids in length;
optionally, the third linker has an amino acid sequence as set forth in SEQ ID NO: 6;
optionally, the fourth linker has an amino acid sequence as set forth in SEQ ID NO: 7.

8. The fused protein according to any one of claims 1 to 7, wherein the fused protein has an amino acid sequence as set forth in SEQ ID NO: 8.

9. A nucleic acid, encoding the fused protein of any one of claims 1 to 8, preferably, the nucleic acid is a nucleic acid subjected to a codon optimization for the yeast expression system; and
more preferably, the nucleic acid has a nucleotide sequence as set forth in SEQ ID NO: 10.

10. An expression vector, comprising the nucleic acid according to claim 9.

11. A recombinant cell, carrying the nucleic acid according to claim 9, the expression vector according to claim 10, or expressing the fused protein according to any one of claims 1 to 8.

12. The fused protein according to any one of claims 1 to 8, the nucleic acid according to claim 9, the expression vector according to claim 10, and the recombinant cell according to claim 11 for use in the treatment or prevention of a tumor or an inflammatory disease or for use in the culture and proliferation of NK cells *in vitro.*

13. A pharmaceutical composition, comprising the fused protein according to any one of claims 1 to 8, the nucleic acid according to claim 9, the expression vector according to claim 10, or the recombinant cell according to claim 11, wherein the pharmaceutical composition is used for treating or preventing a tumor or an inflammatory disease;
optionally, the tumor comprises at least one of ovarian cancer, lung cancer, rectal cancer, breast cancer, or leukemia;
optionally, the inflammatory disease comprises a bacterial or virus infection;
optionally, the bacterial infection comprises at least one of *Klebsiella pneumoniae* infection, *Listeria monocytogenes* infection, or *Staphylococcus aureus* infection; and
optionally, the virus infection comprises at least one of HPV infection, HBV infection, influenza virus infection, or coronavirus infection.

14. A kit, comprising the fused protein of any one of claims 1 to 8, wherein the kit is used for the detection of CD16A and/or 4-1BB.

## Patentansprüche

1. Fusionsprotein, umfassend einen CD16A-Einzelkettenantikörper und eine 4-1BBL-Extrazellulärregion, wobei der CD16A-Einzelkettenantikörper mit der 4-1BBL-Extrazellulärregion verbunden ist, **dadurch gekennzeichnet, dass** die 4-1BBL-Extrazellulärregion drei 4-1BBL-Extrazellulärdomänen umfasst, wobei die drei 4-1BBL-Extrazellulärdomänen sequenziell verbunden sind.

2. Fusionsprotein gemäß Anspruch 1, wobei der CD16A-Einzelkettenantikörper und die 4-1BBL-Extrazellulärregion durch einen flexiblen Linker verbunden sind;
optional wobei die drei 4-1BBL-Extrazellulärdomänen durch den flexiblen Linker verbunden sind.

3. Fusionsprotein nach Anspruch 1 oder 2, wobei das Fusionsprotein durch ein Nicht-Säugerzell-Expressionssystem exprimiert wird;
optional wobei das Nicht-Säugerzell-Expressionssystem mindestens eines von einem prokaryotischen Expressionssystem und einem niedrigstufigen eukaryotischen Expressionssystem umfasst;
optional wobei das Nicht-Säugerzell-Expressionssystem mindestens eines von einem *Escherichia coli* -Expressionssystem und einem Hefe-Expressionssystem umfasst; und
optional wobei das Hefe-Expressionssystem ein *Pichia pastoris* -Expressionssystem umfasst.

4. Fusionsprotein nach einem der Ansprüche 1 bis 3, wobei:
der CD16A-Einzelkettenantikörper eine V_{H}-Region und eine V_{L}-Region umfasst;
die V_{H} -Region eine Aminosäuresequenz gemäß SEQ ID NR: 1 aufweist;
die V_{L} -Region eine Aminosäuresequenz gemäß SEQ ID NR: 2 aufweist;
optional wobei die V_{H} - und V_{L} -Regionen durch einen ersten Linker verbunden sind;
optional wobei der erste Linker ein flexibler Linker ist;
optional wobei der erste Linker 10 bis 20 Aminosäuren in der Länge hat;
optional wobei der erste Linker eine Aminosäuresequenz gemäß SEQ ID NR: 3 aufweist.

5. Fusionsprotein gemäß Anspruch 4, wobei die V_{H} -Region oder die V_{L} -Region an einem Ende durch einen zweiten Linker mit der 4-1BBL-Extrazellulärregion verbunden ist;
optional wobei der zweite Linker ein flexibler Linker ist;
optional wobei der zweite Linker 10 bis 20 Aminosäuren in der Länge hat;
optional wobei der zweite Linker eine Aminosäuresequenz gemäß SEQ ID NR: 4 aufweist.

6. Fusionsprotein nach einem der Ansprüche 1 bis 5, wobei die drei 4-1BBL-Extrazellulärdomänen jeweils eine Aminosäuresequenz gemäß SEQ ID NR: 5 aufweisen.

7. Fusionsprotein nach einem der Ansprüche 1 bis 6, wobei die drei 4-1BBL-Extrazellulärdomänen paarweise durch einen dritten Linker und einen vierten Linker verbunden sind, wobei der dritte Linker und der vierte Linker gleich oder unterschiedlich sind;
optional wobei der dritte Linker und der vierte Linker beide flexible Linker sind;
optional wobei der dritte Linker und der vierte Linker 15 bis 25 Aminosäuren in der Länge haben;
optional wobei der dritte Linker eine Aminosäuresequenz gemäß SEQ ID NR: 6 aufweist;
optional wobei der vierte Linker eine Aminosäuresequenz gemäß SEQ ID NR: 7 aufweist.

8. Fusionsprotein nach einem der Ansprüche 1 bis 7, wobei das Fusionsprotein eine Aminosäuresequenz gemäß SEQ ID NR: 8 aufweist.

9. Nukleinsäure, die das Fusionsprotein nach einem der Ansprüche 1 bis 8 Kodiert, wobei vorzugsweise die Nukleinsäure eine Nukleinsäure ist, die einer Kodonoptimierung des Hefe-Expressionssystems unterzogen wurde; und
mehr bevorzugt wobei die Nukleinsäfure eine Nukleotidsequenz gemäß SEQ ID NR: 10 aufweist.

10. Expressionsvektor, umfassend die Nukleinsäure nach Anspruch 9.

11. Rekombinante Zelle, die die Nukleinsäure nach Anspruch 9 und den Expressionsvektor nach Anspruch 10 trägt oder das Fusionsprotein nach einem der Ansprüche 1 bis 8 exprimiert.

12. Verwendung des Fusionsproteins nach einem der Ansprüche 1 bis 8, der Nukleinsäure nach Anspruch 9, des Expressionsvektors nach Anspruch 10 und der rekombinanten Zelle nach Anspruch 11 zur Behandlung oder Prävention eines Tumors oder einer entzündlichen Erkrankung oder zur Verwendung bei der Kultivierung und Proliferation von NK-Zellen *in vitro.*

13. Pharmazeutische Zusammensetzung, umfassend das Fusionsprotein nach einem der Ansprüche 1 bis 8, die Nukleinsäure nach Anspruch 9, den Expressionsvektor nach Anspruch 10 oder die rekombinante Zelle nach Anspruch 11, wobei die pharmazeutische Zusammensetzung zur Behandlung oder Prävention eines Tumors oder einer entzündlichen Erkrankung verwendet wird;
optional wobei der Tumor mindestens einen aus Eierstockkrebs, Lungenkrebs, Mastdarmkrebs, Brustkrebs oder Leukämie umfasst;
optional wobei die entzündliche Erkrankung eine Bakterieninfektion oder Virusinfektion umfasst;
optional wobei die Bakterieninfektion mindestens eine aus Klebsiella pneumoniae-Infektion, *Listeria monocytogenes-Infektion* oder *Staphylococcus* aureus-Infektion umfasst; und
optional wobei die Virusinfektion mindestens eine aus HPV-Infektion, HBV-Infektion, Influenza-Virus-Infektion oder Coronavirus-Infektion umfasst.

14. Kit, umfassend das Fusionsprotein gemäß einem der Ansprüche 1 bis 8, wobei das Kit zum Nachweis von CD16A und/oder 4-1BB verwendet wird.

## Revendications

1. Protéine fusionnée, comprenant un anticorps simple chaîne anti-CD16A et une région extracellulaire de 4-1BBL, dans laquelle l'anticorps simple chaîne anti-CD16A est lié à la région extracellulaire de 4-1BBL, **caractérisée en ce que** la région extracellulaire de 4-1BBL comprend trois domaines extracellulaires de 4-1BBL, lesdits trois domaines extracellulaires de 4-1BBL étant liés en séquence.

2. Protéine fusionnée selon la revendication 1, dans laquelle l'anticorps simple chaîne anti-CD16A et la région extracellulaire de 4-1BBL sont liés par un lien flexible;
optionnellement, les trois domaines extracellulaires de 4-1BBL sont liés par un lien flexible.

3. Protéine fusionnée selon la revendication 1 ou 2, dans laquelle la protéine fusionnée est exprimée par un système d'expression cellulaire non-mammalien;
optionnellement, le système d'expression cellulaire non-mammalien comprend au moins l'un d'un système d'expression procaryote et d'un système d'expression eucaryote de bas niveau;
optionnellement, le système d'expression cellulaire non-mammalien comprend au moins l'un d'un système d'expression dans *Escherichia coli* et d'un système d'expression dans levure; et
optionnellement, le système d'expression par levure comprend un système d'expression par *Pichia pastoris.*

4. Protéine fusionnée selon l'une quelconque des revendications 1 à 3, dans laquelle:
l'anticorps simple chaîne CD16A comprend une région V_{H} et une région V_{L},
la région V_{H} présente une séquence d'acides aminés telle que définie dans SEQ ID NO: 1;
la région V_{L} présente une séquence d'acides aminés telle que définie dans SEQ ID NO: 2;
optionnellement, les régions V_{H} et V_{L} sont liées par un premier lien;
optionnellement, le premier lien est un lien flexible;
optionnellement, la longueur du premier lien est de 10 à 20 acides aminés;
optionnellement, le premier lien présente une séquence d'acides aminés telle que définie dans SEQ ID NO: 3.

5. Protéine fusionnée selon la revendication 4, dans laquelle une extrémité de la région V_{H} ou de la région V_{L} est liée à la région extracellulaire de 4-1BBL par un deuxième lien;
optionnellement, le deuxième lien est un lien flexible;
optionnellement, la longueur du deuxième lien est de 10 à 20 acides aminés;
optionnellement, le deuxième lien présente une séquence d'acides aminés telle que définie dans SEQ ID NO: 4.

6. Protéine fusionnée selon l'une quelconque des revendications 1 à 5, dans laquelle chacun des trois domaines extracellulaires de 4-1BBL présente une séquence d'acides aminés telle que définie dans SEQ ID NO: 5.

7. Protéine fusionnée selon l'une quelconque des revendications 1 à 6, dans laquelle les trois domaines extracellulaires de 4-1BBL sont liés deux à deux respectivement par un troisième lien et un quatrième lien, le troisième lien et le quatrième lien étant identiques ou différents;
optionnellement, le troisième lien et le quatrième lien sont tous deux des liens flexibles;
optionnellement, la longueur du troisième lien et du quatrième lien est de 15 à 25 acides aminés;
optionnellement, le troisième lien présente une séquence d'acides aminés telle que définie dans SEQ ID NO: 6;
optionnellement, le quatrième lien présente une séquence d'acides aminés telle que définie dans SEQ ID NO: 7.

8. Protéine fusionnée selon l'une quelconque des revendications 1 à 7, dans laquelle la protéine fusionnée présente une séquence d'acides aminés telle que définie dans SEQ ID NO: 8.

9. Acide nucléique, codant pour la protéine fusionnée selon l'une quelconque des revendications 1 à 8, de préférence, l'acide nucléique est un acide nucléique soumis à une optimisation de codons pour le système d'expression par levure; et
plus préférentiellement, l'acide nucléique présente une séquence nucléotidique telle que définie dans SEQ ID NO: 10.

10. Vecteur d'expression, comprenant l'acide nucléique selon la revendication 9.

11. Cellule recombinante, portant l'acide nucléique selon la revendication 9, le vecteur d'expression selon la revendication 10, ou exprimant la protéine fusionnée selon l'une quelconque des revendications 1 à 8.

12. Protéine fusionnée selon l'une quelconque des revendications 1 à 8, acide nucléique selon la revendication 9, vecteur d'expression selon la revendication 10, et cellule recombinante selon la revendication 11, destinés à être utilisés dans le traitement ou la prévention d'un cancer ou d'une maladie inflammatoire, ou destinés à être utilisés dans la culture et la prolifération de cellules NK *in vitro.*

13. Composition pharmaceutique, comprenant la protéine fusionnée selon l'une quelconque des revendications 1 à 8, l'acide nucléique selon la revendication 9, le vecteur d'expression selon la revendication 10, ou la cellule recombinante selon la revendication 11, dans laquelle la composition pharmaceutique est destinée au traitement ou à la prévention d'un cancer ou d'une maladie inflammatoire;
optionnellement, le cancer comprend au moins l'un des cancers suivants: cancer de l'ovaire, cancer du poumon, cancer du rectum, cancer du sein ou leucémie;
optionnellement, la maladie inflammatoire comprend une infection bactérienne ou virale;
optionnellement, l'infection bactérienne comprend au moins l'une des infections suivantes: infection *à Klebsiella pneumoniae,* infection *à Listeria monocytogenes* ou infection *à Staphylococcus aureus;* et
optionnellement, l'infection virale comprend au moins l'une des infections suivantes: infection par le VPH, infection par le VHB, infection par le virus de la grippe ou infection par le coronavirus.

14. Kit, comprenant la protéine fusionnée selon l'une quelconque des revendications 1 à 8, dans lequel le kit est destiné à la détection de CD16A et/ou de 4-1BB.
